# EUROPEAN PATENT APPLICATION

(11) **EP 3 626 251 A1**
(43) Date of publication of application: **25.03.2020**
(21) Application number: 19189181.1
(22) Date of filing: 08.07.2015
(51) Int. Cl.: A61K 38/00, A61P 3/10, C07K 16/40

(54) **COMPOSITIONS AND METHODS FOR TREATING DIABETES**

(30) Priority: 08.07.2014 US 201462021859 P; 04.12.2014 US 201462087566 P
(62) Divisional of application: 15739155.8
(71) Applicant: The Children's Medical Center Corporation, Boston, Massachusetts 02115 (US)
(72) Inventor: Ozcan, Umut, Boston, MA 02130 (US); Herrema, Hilde, 1014 BG Amsterdam (NL)
(74) Representative: Potter Clarkson

(57) **Abstract**

Compositions containing an FKBP 11 peptide (i.e., FKBP11 polypeptide, a variant or a fragment thereof), a fusion protein containing an FKBP11 peptide, or a nucleic acid encoding an FKBP 11 peptide are disclosed.

Also disclosed are methods of reducing blood glucose levels, improving glucose tolerance, decreasing hepatic gluconeogenic activity and/or improving insulin sensitivity in a subject, by administering a composition containing an FKBP 11 peptide or a fusion protein containing an FKBP11 peptide. The methods can include administering nucleic acids encoding an FKBP 11 peptide or a fusion protein containing an FKBP11 peptide, to the subject, or cells expressing the nucleic acids.

Kits containing an FKBP 11 peptide, are also provided.

SEQUENCE LISTING

<110> The Children's Medical Center Corporation

<120> COMPOSITIONS AND METHODS FOR TREATING DIABETES

<130> PABBE/P62555EPdiv1

<150> US 62/021859
<151> 2014-07-08

<150> US 62/087566
<151> 2014-12-04

<160> 10

<170> BiSSAP 1.3.6

<210> 1
<211> 201
<212> PRT
<213> Artificial Sequence

<220>
<223> Synthetic Polypeptide

<400> 1
Met Thr Leu Arg Pro Ser Leu Leu Pro Leu His Leu Leu Leu Leu Leu
1               5                   10                  15
Leu Leu Ser Ala Ala Val Cys Arg Ala Glu Ala Gly Leu Glu Thr Glu
            20                  25                  30
Ser Pro Val Arg Thr Leu Gln Val Glu Thr Leu Val Glu Pro Pro Glu
        35                  40                  45
Pro Cys Ala Glu Pro Ala Ala Phe Gly Asp Thr Leu His Ile His Tyr
    50                  55                  60
Thr Gly Ser Leu Val Asp Gly Arg Ile Ile Asp Thr Ser Leu Thr Arg
65                  70                  75                  80
Asp Pro Leu Val Ile Glu Leu Gly Gln Lys Gln Val Ile Pro Gly Leu
                85                  90                  95
Glu Gln Ser Leu Leu Asp Met Cys Val Gly Glu Lys Arg Arg Ala Ile
            100                 105                 110
Ile Pro Ser His Leu Ala Tyr Gly Lys Arg Gly Phe Pro Pro Ser Val
        115                 120                 125
Pro Ala Asp Ala Val Val Gln Tyr Asp Val Glu Leu Ile Ala Leu Ile
    130                 135                 140
Arg Ala Asn Tyr Trp Leu Lys Leu Val Lys Gly Ile Leu Pro Leu Val
145                 150                 155                 160
Gly Met Ala Met Val Pro Ala Leu Leu Gly Leu Ile Gly Tyr His Leu
                165                 170                 175
Tyr Arg Lys Ala Asn Arg Pro Lys Val Ser Lys Lys Lys Leu Lys Glu
            180                 185                 190
Glu Lys Arg Asn Lys Ser Lys Lys Lys
        195                 200

<210> 2
<211> 726
<212> DNA
<213> Homo sapiens

<400> 2
gaacgagggt cctagctgcc gccacccgaa cagcctgtcc tggtgccccg gctccctgcc      60

ccgcgcccag tcatgaccct gcgcccctca ctcctcccgc tccatctgct gctgctgctg 120

ctgctcagtg cggcggtgtg ccgggctgag gctgggctcg aaaccgaaag tcccgtccgg 180

accctccaag tggagaccct gtggagcccc cagaaccatg tgccgagccc gctgcttttg 240

gagacacgct tcacatacac tacacgggaa gcttggtaga tggacgtatt attgacacct 300

ccctgaccag agaccctctg gttatagaac ttggccaaaa gcaggtgatt ccaggtctgg 360

agcagagtct tctcgacatg tgtgtgggag agaagcgaag ggcaatcatt ccttctcact 420

tggcctatgg aaaacgggga tttccaccat ctgtcccagc ggatgcagtg gtgcagtatg 480

acgtggagct gattgcacta atccgagcca actactggct aaagctggtg aagggcattt 540

tgcctctggt agggatggcc atggtgccag ccctcctggg cctcattggg tatcacctat 600

acagaaaggc caatagaccc aaagtctcca aaaagaagct caaggaagag aaacgaaaca 660

agagcaaaaa gaaataataa ataataaatt ttaaaaaact taaaaaaaaa aaaaaaaaaa 720

aaaaaa 726

<210> 3
<211> 11
<212> PRT
<213> Human immunodeficiency virus

<400> 3
Tyr Gly Arg Lys Lys Arg Arg Gln Arg Arg Arg
1               5                   10

<210> 4
<211> 9
<212> PRT
<213> Human immunodeficiency virus

<400> 4
Arg Lys Lys Arg Arg Gln Arg Arg Arg
1               5

<210> 5
<211> 12
<212> PRT
<213> Artificial Sequence

<220>
<223> Protein transduction domain

<400> 5
Arg Arg Gln Arg Arg Thr Ser Lys Leu Met Lys Arg
1               5                   10

<210> 6
<211> 27
<212> PRT
<213> Artificial Sequence

<220>
<223> Protein transduction domain

<400> 6
Gly Trp Thr Leu Asn Ser Ala Gly Tyr Leu Leu Gly Lys Ile Asn Leu
1               5                   10                  15
Lys Ala Leu Ala Ala Leu Ala Lys Lys Ile Leu
            20                  25

<210> 7
<211> 29
<212> PRT
<213> Artificial Sequence


<220>
<223> Protein transduction domain

<400> 7
Trp Glu Ala Lys Leu Ala Lys Ala Leu Ala Lys Ala Leu Ala Lys His
1               5                   10                  15
Leu Ala Lys Ala Leu Ala Lys Ala Leu Lys Cys Glu Ala
            20                  25

<210> 8
<211> 16
<212> PRT
<213> Artificial Sequence


<220>
<223> Protein transduction domain

<400> 8
Arg Gln Ile Lys Ile Trp Phe Gln Asn Arg Arg Met Lys Trp Lys Lys
1               5                   10                  15



<210> 9
<211> 20
<212> PRT
<213> Artificial Sequence


<220>
<223> Endosomal Escape Sequence

<400> 9
Gly Asp Ile Met Gly Glu Trp Gly Asn Glu Ile Phe Gly Ala Ile Ala
1               5                   10                  15
Gly Phe Leu Gly
            20

<210> 10
<211> 35
<212> PRT
<213> Artificial Sequence


<220>

<223> Protein transduction domain

<400> 10
Arg Arg Arg Arg Arg Arg Arg Arg Arg Arg Arg Gly Glu Gly Asp Ile
1               5                   10                  15
Met Gly Glu Trp Gly Asn Glu Ile Phe Gly Ala Ile Ala Gly Phe Leu
            20                  25                  30
Gly Gly Glu
        35

## Description

### FIELD OF THE INVENTION

The invention is generally related to the field of metabolic homeostasis, more particularly to methods and compositions for lowering blood glucose levels, and treating diabetes.

### BACKGROUND OF THE INVENTION

Diabetes mellitus (DM) is a group of metabolic diseases where the subject has high blood sugar, either because the pancreas does not produce enough insulin, or, because cells do not respond to insulin that is produced. Diabetes affects more than 25.8 million people in the United States alone, i.e. 8.3% of the population. About 1.9 million people aged 20 years or older were newly diagnosed with diabetes in 2010. An estimated 79 million people aged 20 years or older are believed to have prediabetes, which constitutes 5% of adults aged 20 years or older and 50% of adults aged 65 years or older. National Diabetes Information Clearinghouse, National Diabetes Statistics, 2011.

Much of the morbidity and cost of diabetes management is attributable to long-term diabetes-related complications. For example, diabetes is the leading cause of kidney failure, non-traumatic lower limb amputations and new cases of blindness among adults. Diabetes is also a major cause of heart disease and stroke. After adjusting for population age and sex differences, average medical expenditures among people with diagnosed diabetes were 2.3 times higher than the expected expenditures without diabetes. The chronic elevation of blood glucose level associated with DM leads to damage of blood vessels. The resulting problems are grouped under "microvascular disease" (due to damage to small blood vessels) and "macrovascular disease" (due to damage to the arteries). The damage to small blood vessels leads to a microangiopathy, which can cause diabetic retinopathy and/or diabetic nephropathy. Microvascular complications including retinopathy and nephropathy account for the most prevalent and severe morbidity associated with diabetes and may be involved in mediating the increased risk of cardio- and cerebrovascular disease as well. Diabetes is also the leading cause of renal insufficiency and end-stage renal disease (ESRD) in the U.S., and the Western world. Although diabetic microvascular complications are clearly associated with the degree of hyperglycemia, not all diabetic individuals with poor glycemic control develop renal or advanced retinal complications. Conversely, some diabetic patients develop severe complications despite well-controlled blood glucose concentrations.

There are two main types of diabetes. Type 1 diabetes results from the body's failure to produce insulin. Type 2 diabetes results from insulin resistance, a condition in which cells fail to use insulin properly, sometimes combined with an absolute insulin deficiency. This form was previously referred to as non insulin-dependent diabetes mellitus (NIDDM) or "adult-onset diabetes". A third form, gestational diabetes occurs when pregnant women without a previous diagnosis of diabetes develop a high blood glucose level. It may precede development of type 2 diabetes, or it may resolve at the end of the pregnancy.

The cost of diabetes in 2007 was $175 billion, which includes $116 billion in excess medical expenditures and $58 billion in reduced national productivity. Dall, et al., Diabetes Care, 31(3):596-615 (2008). Because patients with Type 1 diabetes produce no insulin, the primary treatment for Type 1 diabetes is daily intensive insulin therapy. The treatment of Type 2 diabetes typically starts with management of diet and exercise. Although helpful in the short-run, treatment through diet and exercise alone is not an effective long-term solution for the vast majority of patients with Type 2 diabetes. When diet and exercise are no longer sufficient, treatment commences with various non-insulin oral medications. These oral medications act by increasing the amount of insulin produced by the pancreas, by increasing the sensitivity of insulin-sensitive cells, by reducing the glucose output of the liver or by some combination of these mechanisms. These treatments are limited in their ability to manage the disease effectively and generally have significant side effects, such as weight gain and hypertension. Because of the limitations of non-insulin treatments, many patients with Type 2 diabetes progress over time and eventually require insulin therapy to support their metabolism. Many of the known hypoglycemic agents exhibit undesirable side effects and are toxic in certain cases. Accordingly, there is a need for additional methods and compositions for treating diabetes.

It is an object of the present invention to compositions for treating diabetes in a subject.

It is also an object of the present of the present invention to provide a method for treating diabetes in a subject.

It is a further object of the invention to provide kits for treating diabetes in a subject.

### SUMMARY OF THE INVENTION

The compositions provided herein are based on the discovery that FK506-binding protein 11 (FKBP 11) plays a role in glucose metabolism. FKBP 11 lowers blood glucose levels, improve glucose tolerance, decreases hepatic gluconeogenic activity and/or insulin sensitivity in a subject.

Compositions containing an effective amount of FKBP 11 peptide can be used to treat a subject diagnosed with type 1 or type 2 diabetes to lower blood glucose levels, improve glucose tolerance, decrease hepatic gluconeogenic activity and/or insulin sensitivity in a subject. The compositions disclosed herein can include nucleic acids encoding FKBP 11 peptide or a fusion protein including an FKBP 11 peptide, vectors containing such nucleic acids and host cells expressing the vectors, either for administration of the nucleic acid to an individual or for expression of protein for administration to an individual. In one embodiment, the host cell is a mammalian cell, preferably a human cell, more preferably, a pancreatic cell or pancreatic progenitor cell. In still other embodiments, the host cell is a yeast cell. In other embodiments the cell is a prokacryotic cell. The host cell may also be used in a screening assay for agents which upregulate/down regulate glucose modulating activities of an FKBP 11 peptide.

Also provided is a method of controlling blood glucose levels, improve glucose tolerance, decrease hepatic gluconeogenic activity and/or insulin sensitivity in a subject, by administering a composition containing an FKBP 11 peptide or a fusion protein including an FKBP 11 peptide. The methods can include administering nucleic acids encoding an FKBP 11 peptide, to the subject. In one embodiment, the nucleic acid is administered *in vivo.* In another embodiment, the nucleic acid is administered *ex vivo,* whereby cells are removed from a subject, and a nucleic acid encoding an FKBP 11 peptide, or a fusion protein including an FKBP 11 peptide, is introduced into the cells, which are then reintroduced into the subject. The subject is preferably a mammal, more preferably, a human subject or an animal subject, for example, domestic animals and pets. The subject can be a type 1 diabetic, a type II diabetic, an obese subject, a subject exhibiting higher than normal blood glucose levels, or a gestational diabetic.

Also provided are kits containing an FKBP 11 peptide or a fusion protein including an FKBP 11 peptide, for treating or alleviating one or more symptoms of diabetes in a subject. The FKBP 11 peptide can be stored in one container and the excipients can be stored in a second container. Immediately prior to administration the contents of both containers are mixed. In one embodiment, the kit may contain a vial containing lyophilized FKBP 11 peptide or a fusion protein including the FKBP 11 peptide, in the cap, separated by a seal which can be broken by rotation of the cap, to allow the insulin to mix with the excipient solution in the vial.

### BRIEF DESCRIPTION OF THE DRAWINGS

Figure 1A shows gene expression levels in livers of genetically obese and diabetic ob/ob mice compared to lean mice. Figure 1B shows gene expression levels in high fat diet (HFD)-induced obese and insulin resistant mice, compared to lean mice.
Figure 2A shows gene expression following overexpression of FKBP11 in livers of lean mice. Figures 2B-2D show body weight (Fig 2B), food intake (Fig. 2C) and blood glucose levels (Fig. 2D) in lean mice injected with FKBP11-containing adenovirus, when compared to lean mice injected with adLacZ (control).
Figure 3A shows gene expression following overexpression of FKBP11 in livers of genetically obese and diabetic ob/ob mice. Figures 3B-3D show the effect of overexpression of FKBP11 in livers of ob/ob mice, on body weight (Figure 3B), food intake (Figure 3C), and blood glucose levels (Figure 3D).

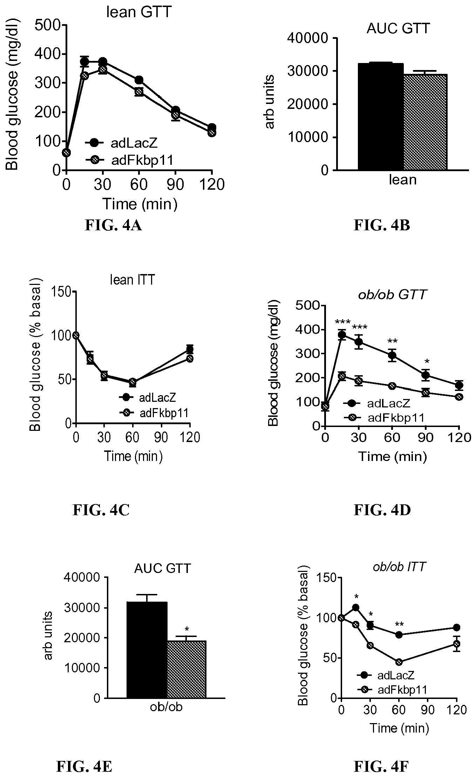

Figure 4A shows the effect of overexpression of FKBP11 on glucose tolerance as assessed by glucose tolerance testing (GTT) in lean mice (area under the curve for Figure 4A is depicted in Figure 4B). Figure 4C shows the effect of overexpression of FKBP11 on insulin tolerance as assessed by means of an insulin tolerance test (ITT). Figure 4D shows the effect of overexpression of FKBP11 on glucose tolerance in ob/ob mice as assessed by GTT (area under the curve for Figure 4D is depicted in Figure 4E). Figure 4F shows the effect of overexpression of FKBP11 on insulin tolerance as assessed by means of an insulin tolerance test (ITT).
Figures 5A-5D show the effect of FKPB11 overexpression on hepatic glucose production as assessed by pyruvate tolerance test (PTT) in lean and obese mice. Figures 5A and 5B show glucose levels and the AUC for PTT, respectively, for lean mice. Figures 5C and 5D show glucose levels as assessed by pyruvate tolerance test (PTT) and AUC for PTT respectively, in ob/ob mice.
Figure 6A shows gene expression levels for FKBP11in HFD-fed mice that overexpress FKBP11. Figures 6B-6E show the effect of FKBP 11 on food intake (Figure 6B), body weight (Figure 6C) and glucose levels (Figure 6D and 6E). AUC for Figure 6E is depicted in Figure 6F.
Figure 7A shows endogenous gene expression levels of FKBP11 in livers of STZ-induced type I diabetic mice. Figures 7B and show hepatic gene and protein expression of STZ-induced type I diabetic mice that overexpress FKBP11. Figures 7C to 7F show the effect of FKBP11 on insulin levels (Figure 7C), body weight (Figure 7D), food intake (Figure 7E), and blood glucose levels (Figure 7F).
Figure 8 shows FKBP11 ELISA read outs (A450nm) from cell culture media of HEK cells overexpressing FKBP11 are presented.
Figure 9 shows blood glucose levels following iv administration of recombinant FKBP11 in diabetic mice, when compared to control (Buffer).

### DETAILED DESCRIPTION OF THE INVENTION

It has been discovered that there is a direct link between low levels of secreted FKBP11 and glucose metabolism. As demonstrated by the examples, FKBP11 is involved in maintaining glucose homeostasis in obese and type 2 diabetic mice, as well as in a mouse model of type 1 diabetes. FKBP11 expression is dynamically regulated in healthy lean mice that are subjected to metabolic stress such as refeeding after a fasting period, indicating an important physiological role in metabolic control. Hepatic expression levels of FKBP11 are reduced in obese and type 2 diabetic mice. Restoring FKBP11 levels dramatically reduced fed and fasted blood glucose levels, and improved glucose tolerance, hepatic gluconeogenic activity and insulin sensitivity. FKBP11 expression also reduced glucose levels in a mouse model of type-I diabetes.

Accordingly, compositions and methods for reducing glucose levels, improving glucose tolerance and improving insulin sensitivity, by increasing FKBP11 peptide in a subject, are provided. The preferred FKBP11 is an FKBP11 polypeptide, represented by SEQ ID NO: 1.

### I. DEFINITIONS

"Effective amount" is used herein to refer to a sufficient amount of an agent to provide a desired effect. The exact amount required will vary from subject to subject, depending on the species, age, and general condition of the subject, the severity of disease that is being treated, the particular agent used, and its mode of administration. An appropriate "effective amount" may be determined empirically by one of ordinary skill in the art using routine methods.
"Expression vector" is used herein to refer to a vector that includes one or more expression control sequences.
"Expression control sequence" is used herein to refer to a DNA sequence that controls and regulates the transcription and/or translation of another DNA sequence.
"FKBP 11" is used herein interchangeably with "FKBP 19". It belongs to a family of proteins known as peptidyl-prolyl *cis*/*trans* isomerases (PPIase) involved in folding of proline-containing polypeptides.
"FKBP11 polypeptide, fragments thereof, variants thereof are collectively referred to herein as "FKBP11 peptides".
"Identity," as known in the art, is a relationship between two or more polypeptide sequences, as determined by comparing the sequences. In the art, "Identity" also means the degree of sequence relatedness between polypeptide as determined by the match between strings of such sequences.
"Insulin resistance" is used herein to refer to a physiological condition in a subject where insulin becomes less effective at lowering blood sugars (low insulin sensitivity), which results in an increase in blood glucose. Insulin resistance in muscle and fat cells reduces glucose uptake, whereas insulin resistance in liver cells results in reduced glycogen synthesis and storage and a failure to suppress glucose production and release into the blood.
"Isolated nucleic acid" is used herein to refer to a nucleic acid that is separated from other nucleic acid molecules that are present in a mammalian genome, including nucleic acids that normally flank one or both sides of the nucleic acid in a mammalian genome. The term "isolated" as used herein with respect to nucleic acids also includes the combination with any non-naturally-occurring nucleic acid sequence, since such non-naturally-occurring sequences are not found in nature and do not have immediately contiguous sequences in a naturally-occurring genome.
"Low stringency" as used herein refers to conditions that permit a polynucleotide or polypeptide to bind to another substance with little or no sequence specificity.
"Pharmaceutically acceptable carrier" as used herein encompasses any of the standard pharmaceutical carriers, such as a phosphate buffered saline solution, water and emulsions such as an oil/water or water/oil emulsion, and various types of wetting agents.
"Protein transduction domain" or "PTD" refers to a polypeptide, polynucleotide, carbohydrate, organic or inorganic compound that facilitates traversing a lipid bilayer, micelle, cell membrane, organelle membrane, or vesicle membrane.
"Purified" and similar terms as used herein relate to the isolation of a molecule or compound in a form that is substantially free (at least 60% free, preferably 75% free, and most preferably 90% free) from other components normally associated with the molecule or compound in a native environment.
The term "treatment" refers to the medical management of a subject with the intent to cure, ameliorate, stabilize, or prevent one more symptoms of a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.
"Transformed" and "transfected" are used herein to encompass the introduction of a nucleic acid (e.g. a vector) into a cell by a number of techniques known in the art.
"Variant" refers to a polypeptide or polynucleotide that differs from a reference polypeptide or polynucleotide, but retains essential properties.
"Vector" as used herein refers to a replicon, such as a plasmid, phage, virus or cosmid, into which another DNA segment may be inserted so as to bring about the replication of the inserted segment. Vectors can be expression vectors.

### II. COMPOSITIONS

Compositions for increasing FKPB11 polypeptide include formulations containing a purified FKPB11 peptide. Compositions for increasing FKPB11 polypeptide also include vectors containing nucleic acid sequences encoding an FKBP11 peptide. FKBP11 peptides include FKBP11 polypeptide, fragments thereof, variants thereof and fusion peptides containing an FKBP11 peptide.

Purified FKBP11 peptides can be obtained by expressing and amplifying a vector containing a tagged (e.g., 6^{∗}HIS) form of FKBP11 in eukaryotic cells (preferred), insect cells or bacteria. Tagged FKBP11 will be expressed the cells and can subsequently be purified from cell lysate or cell culture media by antibody-mediated pull down (the antibody recognizes the tag, which allows for clean and efficient isolation of FKBP11). Since some tags interfere with protein activity/specificity, it is possible to have the tag removed after the isolation and purification process.

Formulations containing an isolated FKBP11 peptide as an active agent also contain one or more pharmaceutically suitable excipients. FKBP11 peptides may be administered in the form of a pharmaceutical composition wherein the FKBP11 is in admixture or mixture with one or more pharmaceutically acceptable carriers, excipients or diluents.

In some embodiments, the FKPB11 peptide may be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

### A. FKBP11 peptides -FKBP11 polypeptides, fragments/ variants thereof, and fusion proteins containing FKBP11

FKBP11 belongs to a family of proteins known as peptidyl-prolyl *cis*/*trans* isomerases (PPIase) involved in folding of proline-containing polypeptides. The PPIase families are classified by sequence homology and pharmacologically by their ability to bind the immunosuppressant compounds cyclosporine, FK506 and rapamycin, and are otherwise known as immunophilins. The FK506-binding protein (FKBP) family shares a high degree of sequence and structural homology and PPIase activity that is specifically inhibited by FK506 or rapamycin. Since the discovery of the first FKBP several members of this family have been characterized in humans and other organisms (Reviewed in Sulten, et al., in Mamm. Genome, 17(4):322-331 (2006).

### 1. FKB11 polypeptide

The human FKBP11 sequence is known (AF238079_1) mtlrpsllpl hlllllllsa avcraeagle tespvrtlqv etlveppepc aepaafgdtl hihytgslvd griidtsltr dplvielgqk qvipgleqsl ldmcvgekrr aiipshlayg krgfppsvpa davvqydvel ialiranywl klvkgilplv gmamvpallg ligyhlyrka nrpkvskkkl keekrnkskk k (SEQ ID NO: 1)

FKBP19 includes a leucine-rich N-terminal leader sequence of 25 residues, which shows similarities with other known secretory pathway proteins. Cleavage at the predicted site of 3 kDa leaves a 19 kDa mature protein, thus named FKBP19. Anti-FKBP19 was used to detect a doublet of 19-22 kDa in bovine pancreas extracts. Immunohistochemical analysis of FKBP19 production in the mouse pancreas shows high levels of FKBP19 protein, localized throughout the cytoplasmic region of acinar cells and concentrated in the perinuclear region of these cells. Low levels are seen in the islets of Langerhans. (Sulten, et al., in Mamm. Genome, 17(4):322-331 (2006)).

FKBP11 has high (around 90%) sequence homology in mice, humans and rats. There are 3 isoforms of FKBP11 predicted in humans. The first domain is a signal peptide, which targets FKBP11 to the secretory pathway. This domain is predicted to be cleaved after AA 25. The second domain is predicted to be a peptidylprolyl isomerase (PPIase) domain, which potentially serves as enzymatic domain. The PPIase domain is highly conserved amongst the FKBP protein family members and for some, but not all, of the FKBP family members, their function is determined by PPIase activity. The third domain is a hydrophobic domain that is predicted to be a transmembrane domain. Similar hydrophobic sequences are found in type I transmembrane protein family of proteins and accordingly, FKBP11 can be categorized as such. Some of these proteins are known to have a cleavage site near the hydrophobic domain that following cleavage release a soluble fragment leaving the transmembrane domain residing in the membrane.

### 2. Variants/fragments of the FKBP11 polypeptide

A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more modifications (e.g., substitutions, additions, and/or deletions). A substituted or inserted amino acid residue may or may not be one encoded by the genetic code. A variant of a polypeptide may be naturally occurring such as an allelic variant, or it may be a variant that is not known to occur naturally.

Modifications and changes can be made in the structure of the polypeptides disclosed herein and still obtain a molecule having similar characteristics as the polypeptide (e.g., a conservative amino acid substitution). For example, certain amino acids can be substituted for other amino acids in a sequence, without appreciable loss of activity. Since it is the interactive capacity and nature of a polypeptide that defines that polypeptide's biological functional activity, certain amino acid sequence substitutions can be made in a polypeptide sequence and nevertheless obtain a polypeptide with like properties.

In making such changes, the hydropathic index of amino acids can be considered. The importance of the hydropathic amino acid index in conferring interactive biologic function on a polypeptide is generally understood in the art. It is known that certain amino acids can be substituted for other amino acids having a similar hydropathic index or score and still result in a polypeptide with similar biological activity. Each amino acid has been assigned a hydropathic index on the basis of its hydrophobicity and charge characteristics. Those indices are: isoleucine (+4.5); valine (+4.2); leucine (+3.8); phenylalanine (+2.8); cysteine/cysteine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is believed that the relative hydropathic character of the amino acid determines the secondary structure of the resultant polypeptide, which in turn defines the interaction of the polypeptide with other molecules, such as enzymes, substrates, receptors, antibodies, and antigens. It is known in the art that an amino acid can be substituted by another amino acid having a similar hydropathic index and still obtain a functionally equivalent polypeptide. In such changes, the substitution of amino acids whose hydropathic indices are within ± 2 is preferred, those within ± 1 are particularly preferred, and those within ± 0.5 are even more particularly preferred.

Substitution of like amino acids can also be made on the basis of hydrophilicity, particularly when the biological functional equivalent polypeptide or peptide thereby created is intended for use in immunological embodiments. The following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamnine (+0.2); glycine (0); proline (-0.5 ± 1); threonine (-0.4); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent polypeptide. In such changes, the substitution of amino acids whose hydrophilicity values are within ± 2 is preferred, those within ± 1 are particularly preferred, and those within ± 0.5 are even more particularly preferred.

Amino acid substitutions are generally based on the relative similarity of the amino acid side-chain substituents, for example, their hydrophobicity, hydrophilicity, charge, and size. Exemplary substitutions that take various of the foregoing characteristics into consideration are well known to those of skill in the art and include (original residue: exemplary substitution): (Ala: Gly, Ser), (Arg: Lys), (Asn: Gln, His), (Asp: Glu, Cys, Ser), (Gln: Asn), (Glu: Asp), (Gly: Ala), (His: Asn, Gln), (Ile: Leu, Val), (Leu: Ile, Val), (Lys: Arg), (Met: Leu, Tyr), (Ser: Thr), (Thr: Ser), (Tip: Tyr), (Tyr: Trp, Phe), and (Val: Ile, Leu). The polypeptides can include variants having about 50%, 60%, 70%, 80%, 90%, and 95% sequence identity to the polypeptide of interest.

"Identity" and "similarity" can be readily calculated by known methods, such as those described in (Computational Molecular Biology, Lesk, A. M., Ed., Oxford University Press, New York, 1988; Biocomputing: Informatics and Genome Projects, Smith, D. W., Ed., Academic Press, New York, 1993; Computer Analysis of Sequence Data, Part I, Griffin, A. M., and Griffin, H. G., Eds., Humana Press, New Jersey, 1994; Sequence Analysis in Molecular Biology, von Heinje, G., Academic Press, 1987; and Sequence Analysis Primer, Gribskov, M. and Devereux, J., Eds., M Stockton Press, New York, 1991; and Carillo and Lipman, SIAM J Applied Math, 48: 1073 (1988).

Preferred methods to determine identity are designed to give the largest match between the sequences tested. Methods to determine identity and similarity are codified in publicly available computer programs. The percent identity between two sequences can be determined by using analysis software (i.e., Sequence Analysis Software Package of the Genetics Computer Group, Madison Wis.) that incorporates the Needelman and Wunsch, (J. Mol. Biol., 48: 443-453, 1970) algorithm (e.g., NBLAST, and XBLAST). The default parameters are used to determine the identity for the polypeptides of the present disclosure.

By way of example, a polypeptide sequence may be identical to the reference sequence, that is be 100% identical, or it may include up to a certain integer number of amino acid alterations as compared to the reference sequence such that the % identity is less than 100%. Such alterations include at least one amino acid deletion, substitution, including conservative and non-conservative substitution, or insertion, wherein the alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in the reference polypeptide by the numerical percent of the respective percent identity (divided by 100) and then subtracting that product from the total number of amino acids in the reference polypeptide.

### 3. Fusion Proteins containing FKBP11 peptides

Fusion proteins, also known as chimeric proteins, are proteins created through the joining of two or more genes which originally coded for separate proteins. Translation of this fusion gene results in a single polypeptide with function properties derived from each of the original proteins. Recombinant fusion proteins can be created artificially by recombinant DNA technology for use in biological research or therapeutics. Chimeric mutant proteins occur naturally when a large-scale mutation, typically a chromosomal translocation, creates a novel coding sequence containing parts of the coding sequences from two different genes.

The FKBP11 peptides disclosed herein can be engineered delivered to a host as a fusion protein, which includes additional domains such as a targeting domain.

The functionality of fusion proteins is made possible by the fact that many protein functional domains are modular. In other words, the linear portion of a polypeptide which corresponds to a given domain, such as a tyrosine kinase domain, may be removed from the rest of the protein without destroying its intrinsic enzymatic capability. Thus, any of the herein disclosed functional domains can be used to design a fusion protein.

A recombinant fusion protein is a protein created through genetic engineering of a fusion gene. This typically involves removing the stop codon from a cDNA sequence coding for the first protein, then appending the cDNA sequence of the second protein in frame through ligation or overlap extension PCR. That DNA sequence will then be expressed by a cell as a single protein. The protein can be engineered to include the full sequence of both original proteins, or only a portion of either.

If the two entities are proteins, often linker (or "spacer") peptides are also added which make it more likely that the proteins fold independently and behave as expected. Especially in the case where the linkers enable protein purification, linkers in protein or peptide fusions are sometimes engineered with cleavage sites for proteases or chemical agents which enable the liberation of the two separate proteins. This technique is often used for identification and purification of proteins, by fusing a GST protein, FLAG peptide, or a hexa-his peptide (aka: a 6xhis-tag) which can be isolated using nickel or cobalt resins (affinity chromatography). Chimeric proteins can also be manufactured with toxins or anti-bodies attached to them in order to study disease development.

Alternatively, internal ribosome entry sites (IRES) elements can be used to create multigene, or polycistronic, messages. IRES elements are able to bypass the ribosome scanning model of 5' methylated Cap dependent translation and begin translation at internal sites (Pelletier and Sonenberg, 1988). IRES elements from two members of the picornavirus family (polio and encephalomyocarditis) have been described (Pelletier and Sonenberg, 1988), as well an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (U.S. Pat. Nos. 5,925, 565 and 5,935,819; PCT/US99/05781). IRES sequences are known in the art and include those from encephalomycarditis virus (EMCV) (Ghattas, et al., Mol. Cell. Biol., 11:5848-5849 (1991); BiP protein (Macejak and Sarnow, Nature, 353:91 (1991)); the Antennapedia gene of drosophilia (exons d and e) [Oh et al., Genes & Development, 6:1643-1653 (1992)); those in polio virus [Pelletier and Sonenberg, Nature, 334:320325 (1988); see also Mountford and Smith, TIG, 11:179-184 (1985)).

### i. Protein transduction domain (PTD)

In some embodiments, the polynucleotide-binding polypeptide is fusion protein modified to include a protein transduction domain (PTD). A PTD attached to another molecule facilitates the molecule traversing membranes, for example going from extracellular space to intracellular space, or cytosol to within an organelle.

In preferred embodiments, the protein transduction domain is a polypeptide. A protein transduction domain can be a polypeptide including positively charged amino acids. Thus, some embodiments include PTDs that are cationic or amphipathic. Protein transduction domains (PTD), also known as a cell penetrating peptides (CPP), are typically polypeptides including positively charged amino acids. PTDs are known in the art, and include but are not limited to small regions of proteins that are able to cross a cell membrane in a receptor-independent mechanism (Kabouridis, P., Trends in Biotechnology (11):498-503 (2003)). Although several PTDs have been documented, the two most commonly employed PTDs are derived from TAT (Frankel and Pabo, Cell, 55(6):1189-93(1988)) protein of HIV and Antennapedia transcription factor from Drosophila, whose PTD is known as Penetratin (Derossi et al., J Biol Chem., 269(14):10444-50 (1994)). Exemplary protein transduction domains include polypeptides with 11 Arginine residues, or positively charged polypeptides or polynucleotides having 8-15 residues, preferably 9-11 residues.

The Antennapedia homeodomain is 68 amino acid residues long and contains four alpha helices. Penetratin is an active domain of this protein which consists of a 16 amino acid sequence derived from the third helix of Antennapedia. TAT protein consists of 86 amino acids and is involved in the replication of HIV-1. The TAT PTD consists of an 11 amino acid sequence domain (residues 47 to 57; YGRKKRRQRR R (SEQ ID NO:3)) of the parent protein that appears to be critical for uptake. Additionally, the basic domain Tat(49-57) or RKKRRQRRR (SEQ ID NO:4) has been shown to be a PTD. In the current literature TAT has been favored for fusion to proteins of interest for cellular import. Several modifications to TAT, including substitutions of Glutatmine to Alanine, i.e., Q→ A, have demonstrated an increase in cellular uptake anywhere from 90% (Wender et al., Proc Natl Acad Sci USA., 97(24):13003-8 (2000)) to up to 33 fold in mammalian cells. (Ho et al., Cancer Res., 61(2):474-7 (2001)).

The most efficient uptake of modified proteins was revealed by mutagenesis experiments of TAT-PTD, showing that an 11 arginine stretch was several orders of magnitude more efficient as an intercellular delivery vehicle. Therefore, PTDs can include a sequence of multiple arginine residues, referred to herein as poly-arginine or poly-ARG. In some embodiments the sequence of arginine residues is consecutive. In some embodiments the sequence of arginine residues is non-consecutive. A poly-ARG can include at least 7 arginine residues, more preferably at least 8 arginine residues, most preferably at least 11 arginine residues. In some embodiments, the poly-ARG includes between 7 and 15 arginine residues, more preferably between 8 and 15 arginine residues. In some embodiments the poly-ARG includes between 7 and 15, more preferably between 8 and 15 consecutive arginine residues. An example of a poly-ARG is RRRRRRR (SEQ ID NO:9). Additional exemplary PTDs include but are not limited to; RRQRRTSKLM KR (SEQ ID NO:5); GWTLNSAGYL LGKINLKALA ALAKKIL (SEQ ID NO:6); WEAKLAKALA KALAKHLAKA LAKALKCEA (SEQ ID NO:7); and RQIKIWFQNR RMKWKK (SEQ ID NO:8).

It is believed that following an initial ionic cell-surface interaction, some polypeptides containing a protein transduction domain are rapidly internalized by cells via lipid raft-dependent macropinocytosis. For example, transduction of a TAT-fusion protein was found to be independent of interleukin-2 receptor/raft-, caveolar- and clathrin-mediated endocytosis and phagocytosis (Wadia, et al., Nature Medicine, 10:310-315 (2004), and Barka, et al., J. Histochem. Cytochem., 48(11):1453-60 (2000)). Therefore, in some embodiments the polynucleotide-binding polypeptide includes an endosomal escape sequence that enhances escape of the polypeptide-binding protein from macropinosomes. The endosomal escape sequence is part of, or consecutive with, the protein transduction domain. In some embodiments, the endosomal escape sequence is non-consecutive with the protein transduction domain. In some embodiments the endosomal escape sequence includes a portion of the hemagglutinin peptide from influenza (HA). One example of an endosomal escape sequence includes GDIMGEWG NEIFGAIAGF LG (SEQ ID NO:9).

In one embodiment a protein transduction domain including an endosomal escape sequence includes the amino acid sequence RRRRRRRRRR RGEGDIMGEW GNEIFGAIAG FLGGE (SEQ ID NO:10).

### ii. Targeting Signal or Domain

In some embodiments the polynucleotide-binding polypeptide is modified to include one or more targeting signals or domains. The targeting signal can include a sequence of monomers that facilitates *in vivo* localization of the molecule. The monomers can be amino acids, nucleotide or nucleoside bases, or sugar groups such as glucose, galactose, and the like which form carbohydrate targeting signals. Targeting signals or sequences can be specific for a host, tissue, organ, cell, organelle, non-nuclear organelle, or cellular compartment. For example, in some embodiments the polynucleotide-binding polypeptide includes both a cell-specific targeting domain and an organelle specific targeting domain to enhance delivery of the polypeptide to a subcellular organelle of a specific cells type.

### B. Nucleic Acids Encoding FKBP11 peptides

Nucleic acids encoding the FKBP 11 polypeptide are known in the art ((accession number AF238079). An FKBP19 (i.e., FKBP11) encoding nucleic acid was characterized by Sulten, et al., in Mamm. Genome, 17(4):322-331 (2006). The 727 bp human FKBP19 mRNA (SEQ ID NO: 2) sequence is derived from 6 exons on chromosome 12. gaacgagggt cctagctgcc gccacccgaa cagcctgtcc tggtgccccg gctccctgcc ccgcgcccag tcatgaccct gcgcccctca ctcctcccgc tccatctgct gctgctgctg ctgctcagtg cggcggtgtg ccgggctgag gctgggctcg aaaccgaaag tcccgtccgg accctccaag tggagaccct gtggagccc ccagaaccat gtgccgagcc cgctgctttt ggagacacgc ttcacataca ctacacggga agcttggtag atggacgtat tattgacacc tccctgacca gagaccctct ggttatagaa cttggccaaa agcaggtgat tccaggtctg gagcagagtc ttctcgacat gtgtgtggga gagaagcgaa gggcaatcat tccttctcac ttggcctatg gaaaacgggg atttccacca tctgtcccag cggatgcagt ggtgcagtat gacgtggagc tgattgcact aatccgagcc aactactggc taaagctggt gaagggcatt ttgcctctgg tagggatggc catggtgcca gccctcctgg gcctcattgg gtatcaccta tacagaaagg ccaatagacc caaagtctcc aaaaagaagc tcaaggaaga gaaacgaaac aagagcaaaa agaaataata aataataaat tttaaaaaac ttaaaaaaaa aaaaaaaaaa aaaaaaa (SEQ ID NO: 2).

In some embodiments, nucleic acids are expressed in cells to produce recombinant FKBP19. In some embodiments the nucleic acid molecules themselves are used in the composition. The compositions can be used in *ex vivo* and *in vivo* methods of gene therapy to increase expression of an active form of an FKBP11 polypeptide, a variant or a fragment thereof.

An isolated nucleic acid can be, for example, a DNA molecule, provided one of the nucleic acid sequences normally found immediately flanking that DNA molecule in a naturally-occurring genome is removed or absent. Thus, an isolated nucleic acid includes, without limitation, a DNA molecule that exists as a separate molecule independent of other sequences (e.g., a chemically synthesized nucleic acid, or a cDNA or genomic DNA fragment produced by PCR or restriction endonuclease treatment), as well as recombinant DNA that is incorporated into a vector, an autonomously replicating plasmid, a virus (e.g., a retrovirus, lentivirus, adenovirus, or herpes virus), or into the genomic DNA of a prokaryote or eukaryote. In addition, an isolated nucleic acid can include an engineered nucleic acid such as a recombinant DNA molecule that is part of a hybrid or fusion nucleic acid. A nucleic acid existing among hundreds to millions of other nucleic acids within, for example, a cDNA library or a genomic library, or a gel slice containing a genomic DNA restriction digest, is not to be considered an isolated nucleic acid.

Nucleic acids encoding active FKBP11 peptides may be optimized for expression in a host. Codons may be substituted with alternative codons encoding the same amino acid to account for differences in codon usage between the organism from which the FKBP11 nucleic acid sequence is derived and the expression host. In this manner, the nucleic acids may be synthesized using expression host-preferred codons. Nucleic acids can be in sense or antisense orientation, or can be complementary to a reference sequence encoding an FKBP11 peptide. Nucleic acids can be DNA, RNA, or nucleic acid analogs. Nucleic acid analogs can be modified at the base moiety, sugar moiety, or phosphate backbone. Such modification can improve, for example, stability, hybridization, or solubility of the nucleic acid. Modifications at the base moiety can include deoxyuridine for deoxythymidine, and 5-methyl-2'-deoxycytidine or 5-bromo-2'-deoxycytidine for deoxycytidine. Modifications of the sugar moiety can include modification of the 2' hydroxyl of the ribose sugar to form 2'-O-methyl or 2'-O-allyl sugars. The deoxyribose phosphate backbone can be modified to produce morpholino nucleic acids, in which each base moiety is linked to a six membered, morpholino ring, or peptide nucleic acids, in which the deoxyphosphate backbone is replaced by a pseudopeptide backbone and the four bases are retained. See, for example, Summerton and Weller (1997) Antisense Nucleic Acid Drug Dev. 7:187-195; and Hyrup et al. (1996) Bioorgan. Med. Chem. 4:5-23. In addition, the deoxyphosphate backbone can be replaced with, for example, a phosphorothioate or phosphorodithioate backbone, a phosphoroamidite, or an alkyl phosphotriester backbone.

Nucleic acids, encoding FKBP11 peptides can be inserted into vectors for expression in a host cell. In some embodiments the host cell is a mammalian cell. In other embodiments, the host can be a prokaryotic cell. The vectors can be used for production of recombinant protein, or in methods of gene therapy. Host cells (e.g., a prokaryotic cell or a eukaryotic cell such as a CHO cell) can be used to, for example, produce the FKBP11 peptides described herein. In some embodiments for *in vivo* transplantation, the host cell is preferably a pancreatic cell or progenitor cell, for example, islet/β-cells of the pancreas.

Nucleic acids in vectors can be operably linked to one or more expression control sequences. For example, the control sequence can be incorporated into a genetic construct so that expression control sequences effectively control expression of a coding sequence of interest. Examples of expression control sequences include promoters, enhancers, and transcription terminating regions. A promoter is an expression control sequence composed of a region of a DNA molecule, typically within 100 nucleotides upstream of the point at which transcription starts (generally near the initiation site for RNA polymerase II). To bring a coding sequence under the control of a promoter, it is necessary to position the translation initiation site of the translational reading frame of the polypeptide between one and about fifty nucleotides downstream of the promoter. Enhancers provide expression specificity in terms of time, location, and level. Unlike promoters, enhancers can function when located at various distances from the transcription site. An enhancer also can be located downstream from the transcription initiation site. A coding sequence is "operably linked" and "under the control" of expression control sequences in a cell when RNA polymerase is able to transcribe the coding sequence into mRNA, which then can be translated into the protein encoded by the coding sequence.

Methods of making vectors for introduction into a cell of choice are known in the art. Suitable expression vectors include, without limitation, plasmids and viral vectors derived from, for example, bacteriophage, baculoviruses, tobacco mosaic virus, herpes viruses, cytomegalo virus, retroviruses, vaccinia viruses, adenoviruses, and adeno-associated viruses. Numerous vectors and expression systems are commercially available from such corporations as Novagen (Madison, WI), Clontech (Palo Alto, CA), Stratagene (La Jolla, CA), and Invitrogen Life Technologies (Carlsbad, CA).

Vectors can be created using molecular cloning and Gateway technology (Life Technologies) according to manufacturers protocol. Vectors contain a promoter derived from cytomegalo virus (CMV). Crude adenovirus are produced by us using ViraPower Adenoviral Gateway Expression Kit (Life Technologies) according to manufacturer's protocol. Crude adenovirus produced by using this method, can be amplified and purified (to obtain pure/clean and highly concentrated adenovirus suitable for injection into mice) by Vector Biolabs (Philadelphia). Mice were injected intravenously (iv) with adenovirus diluted in sterile saline via the tail vein.

Callejas, et al., describe treatment of diabetic dogs by gene therapy, using a one-time intramuscular administration of adeno-associated viral vector. Callehas, Diabetes, Feb 1, 2013, epub ahead of print. Other studies showing successful use of vectors to delivery genes in humans include Morgan, et al., Science, 314(5796):126-9 (2006) (describe conferring tumor recognition by autologous lymphocytes from peripheral blood by using a retrovirus that encodes a T cell receptor); Levine, et al, Proc. natl. Acad. Sci., 103(46):17372-7 (2006) describe lentiviral vectors that can be used for gene transfer to humans.

Vectors containing nucleic acids to be expressed can be transferred into host cells. Although not limited to a particular technique, a number of these techniques are well established within the art. In some embodiments for *in vivo* transplantation, the host cell is preferably a pancreatic cell or progenitor cell, for example, islet cells/β-cells of the pancreas. Methods for isolating host cells, for example, islet cells, are known in the art and are described for example in U.S. Publication No. 2009/0191608. Methods for *in vitro* transfection and *in vivo* transfer of islet cells to a subject, as well as methods for protecting in vivo islet grafts are known in art. (Reviewed in Ajit, et al. Pharmacological reviews, 58(2):194-243 (2006). See also, U.S. Published Application Nos. 2005/0048040, 2011/0008343 and 2011/0182979.

### C. Compounds modifying FKBP11 Activity

FKBP11 is involved in maintaining glucose homeostasis in obese and type 2 diabetic mice, as well as in a mouse model of type 1 diabetes. FKBP11 expression is dynamically regulated in healthy lean mice that are subjected to metabolic stress such as refeeding after a fasting period, indicating an important physiological role in metabolic control. Hepatic expression levels of FKBP11 are reduced in obese and type 2 mice. The examples show that restoring FKBP11 levels dramatically reduced fed and fasted blood glucose levels, and improved glucose tolerance, hepatic gluconeogenic activity and insulin sensitivity. FKBP11 expression also reduced glucose levels in a mouse model of type-I diabetes.

Accordingly, compounds which increase FKBP11 levels or activity or otherwise decrease ER stress through this pathway can be used to maintain or enhance glucose homeostasis, glucose tolerance, hepatic gluconeogenic activity and decrease insulin sensitivity.

Compounds which may be useful in elevating FKBP11 activity or levels and thereby improving glucose homeostasis, may be identified using a variety of known methods, including the animal models described in the examples.

### D. Dosage Forms

Pharmaceutical compositions containing the FKBP11 peptides may be administered parenterally to subjects in need of such a treatment. Parenteral administration can be performed by subcutaneous, intramuscular or intravenous injection by means of a syringe, optionally, a pen-like syringe. Alternatively, parenteral administration can be performed by means of an infusion pump. Alternatively, the peptides are administered orally, nasally or pulmonally, preferably in compositions, powders or liquids, specifically designed for the purpose.

The peptides or nucleic acids described herein can be formulated for parenteral administration. Parenteral formulations can be prepared as aqueous compositions using techniques is known in the art. Typically, such compositions are prepared as injectable formulations, for example, solutions or suspensions; solid forms suitable for using to prepare solutions or suspensions upon the addition of a reconstitution medium prior to injection; emulsions, such as water-in-oil (w/o) emulsions, oil-in-water (o/w) emulsions, and microemulsions thereof, liposomes, or emulsomes. The carrier can be a solvent or dispersion medium containing, for example, water, ethanol, one or more polyols (e.g., glycerol, propylene glycol, and liquid polyethylene glycol), oils, such as vegetable oils (e.g., peanut oil, corn oil, sesame oil, etc.), and combinations thereof.

The parenteral formulations can be formulated for controlled release including immediate release, delayed release, extended release, pulsatile release, and combinations thereof. For example, the compounds and/or one or more additional active agents can be incorporated into polymeric microparticles which provide controlled release of the drug(s). Release of the drug(s) is controlled by diffusion of the drug(s) out of the microparticles and/or degradation of the polymeric particles by hydrolysis and/or enzymatic degradation. Suitable polymers include ethylcellulose and other natural or synthetic cellulose derivatives. Polymers which are slowly soluble and form a gel in an aqueous environment, such as hydroxypropyl methylcellulose or polyethylene oxide may also be suitable as materials for drug containing microparticles. Other polymers include, but are not limited to, polyanhydrides, poly(ester anhydrides), polyhydroxy acids, such as polylactide (PLA), polyglycolide (PGA), poly(lactide-co-glycolide) (PLGA), poly-3-hydroxybutyrate (PHB) and copolymers thereof, poly-4-hydroxybutyrate (P4HB) and copolymers thereof, polycaprolactone and copolymers thereof, and combinations thereof.

Pharmaceutical compositions may be formulated in a conventional manner using one or more physiologically acceptable carriers comprising excipients and auxiliaries which facilitate processing of the active compounds into preparations which can be used pharmaceutically. Formulation of drugs is discussed in, for example, Hoover, John E., Remington's Pharmaceutical Sciences, Mack Publishing Co., Easton, Pa. (1975), and Liberman, H. A. and Lachman, L., Eds., Pharmaceutical Dosage Forms, Marcel Decker, New York, N.Y. (1980). Proper formulation is dependent upon the route of administration chosen.

Pharmaceutically acceptable excipients that can be present in the FKBP11peptide-containing dosage forms include, but are not limited to, diluents, binders, lubricants, disintegrants, colorants, stabilizers, and surfactants. If desired, the tablets, wafers, films, lozenges, beads, granules, or particles may also contain minor amount of nontoxic auxiliary substances such as dyes, sweeteners, coloring and flavoring agents, pH buffering agents, or preservatives.

Solutions and dispersions of the active compounds as the free acid or base or pharmacologically acceptable salts thereof can be prepared in water or another solvent or dispersing medium suitably mixed with one or more pharmaceutically acceptable excipients including, but not limited to, surfactants, dispersants, emulsifiers, pH modifying agents, and combination thereof. In one embodiment, a subcutaneous injectable formulation is produced by mixing an FKBP11 peptide with saline to form a solution and sterilizing the solution (referred to as the "diluent"). The FKBP11 peptide is separately added to sterile water to form a solution, filtered, and a designated amount is placed into each of a number of separate sterile injection bottles. The FKBP11 peptide solution may be lyophilized to form a powder which can be stored separately from the diluent to retain its stability. Prior to administration, the diluent is added to the FKBP11 peptide injection bottle.

The formulation is typically buffered to a pH of 3-8 for parenteral administration upon reconstitution. Suitable buffers include, but are not limited to, phosphate buffers, acetate buffers, and citrate buffers

Water soluble polymers are often used in formulations for parenteral administration. Suitable water-soluble polymers include, but are not limited to, polyvinylpyrrolidone, dextran, carboxymethylcellulose, and polyethylene glycol.

Alternatively, the FKBP11 peptides can be incorporated into microparticles prepared from materials which are insoluble in aqueous solution or slowly soluble in aqueous solution, but are capable of degrading within the GI tract by means including enzymatic degradation, surfactant action of bile acids, and/or mechanical erosion. As used herein, the term "slowly soluble in water" refers to materials that are not dissolved in water within a period of 30 minutes. Preferred examples include fats, fatty substances, waxes, wax-like substances and mixtures thereof. Suitable fats and fatty substances include fatty alcohols (such as lauryl, myristyl stearyl, cetyl or cetostearyl alcohol), fatty acids and derivatives, including, but not limited to, fatty acid esters, fatty acid glycerides (mono-, di- and triglycerides), and hydrogenated fats. Specific examples include, but are not limited to hydrogenated vegetable oil, hydrogenated cottonseed oil, hydrogenated castor oil, hydrogenated oils available under the trade name Sterotex®, stearic acid, cocoa butter, and stearyl alcohol. Suitable waxes and wax-like materials include natural or synthetic waxes, hydrocarbons, and normal waxes. Specific examples of waxes include beeswax, glycowax, castor wax, carnauba wax, paraffins and candelilla wax. As used herein, a wax-like material is defined as any material which is normally solid at room temperature and has a melting point of from about 30 to 300°C.

### III. KITS

The FKBP 11 peptide or a fusion protein containing the FKBP11-peptide can be provided in a kit for use in treating a subject with diabetes. Kits can include one or more containers containing a pharmaceutical composition including a therapeutically effective amount of a specific activator of an FKBP 11 polypeptide, a variant or a fragment therof. Such kits can further include, if desired, one or more of various conventional pharmaceutical kit components, such as, for example, containers with one or more pharmaceutically acceptable carriers as will be readily apparent to those skilled in the art. The kit may also include means of administration, such as one or more of a syringe (e.g., a barrel syringe or a bulb syringe), intravenous (IV) bag, IV line, IV needle, and/or cannula. Printed instructions, either as inserts or as labels, indicating quantities of the components to be administered, guidelines for administration, and/or guidelines for mixing the components, can also be included in the kit.

The FKBP 11 peptide can be stored in one container and the excipients can be stored in a second container. Immediately prior to administration the contents of both containers are mixed.

In one embodiment, the kit may contain a vial containing powdered peptide in the cap, separated by a seal which can be broken by rotation of the cap, to allow the insulin to mix with the excipient solution in the vial.

### IV. METHODS OF USING THE COMPOSITIONS

The compositions described herein are administered to a subject to lower blood glucose levels, to improve glucose tolerance, decrease hepatic gluconeogenic activity and/or insulin sensitivity in the subject. The subject is preferably a mammal, more preferably, a human subject. Representative subjects include type 1 diabetics, type II diabetics, obese subjects, subjects exhibiting higher than normal blood glucose levels, and gestational diabetics.

Normal fasting glucose levels are generally less than about 110 mg/dL. Shortly after eating, the blood glucose level may rise temporarily up to 140 mg/dL. Fasting blood glucose levels over 126 mg/dL, and plasma glucose 2 hours after eating over 200 mg/dL, are indicative of metabolic disorders, such as type-2 diabetes. Therefore, in preferred embodiments, the pharmaceutical compositions are administered in amounts effective to reduce fasting blood glucose levels in the subject to less than 130 mg/dL, preferably less than 110 mg/dL, and/or the plasma glucose 2 hours after eating to less than 200 mg/dL, preferably less than 140 mg/dL.

Efficacy of the disclosed methods can be monitored by measuring changes in blood glucose levels, glucose tolerance, hepatic gluconeogenic, and/or insulin sensitivity content. A statistically significant change in any of these parameters can be considered evidence of therapeutic efficacy. It is preferred that a given marker change by at least 5%, at least 10%, at least 20%, at least 30%, at least 50% or more in effective therapy. Dosage of the pharmaceutical compositions can be modified by the physician to increase efficacy while avoiding side effects or toxicity.

The formulations containing an FKBP11 peptide, nucleic acid molecules encoding the FKBP11 peptide, or compound increasing the activity or levels of an FKBP11 peptide, will be administered in an appropriate vehicle and route for the compound to be delivered, for example, via injection (intravenous, intramuscular, intraperitoneally), topically to a mucosal surface (ocularly, pulmonary, nasal, buccal, rectal or sublingual), or orally.

Nucleic acids encoding an FKBP11 peptide can be administered to subjects in need thereof. Nucleic delivery involves introduction of "foreign" nucleic acids into a cell and ultimately, into a live animal. *In vivo* methods permit direct introduction of the gene therapy agent into the body. *Ex vivo* methods are where certain cells are removed from a human, the gene therapy agent introduced and the cells returned into the body. Methods which are well known to those skilled in the art may be used to construct expression vectors containing sequences encoding polypeptides of interest and appropriate transcriptional and translational control elements. These methods include *in vitro* recombinant DNA techniques, synthetic techniques, and in vivo genetic recombination. Such techniques are described in Sambrook et al., Molecular Cloning, A Laboratory Manual (Cold Spring Harbor Press, 4th ed. Plainview, N.Y., 2012)).

Compositions and methods for delivering nucleic acids to a subject or cell are known in the art (see U.S. Publication Nos. 2014/0065204, 2014/0073053; U.S. Patent No. 7,807618; Li, et al., Pharm Res., 24(3:438-49 (2007); Grigsby, et al., Scientific Reports, 2013 Nov 6;3:3155. doi: 10.1038/srep03155.

One approach of delivering the nucleic acids disclosed herein includes nucleic acid transfer into primary cells in culture followed by autologous transplantation of the *ex vivo* transformed cells into the host, either systemically or into a particular organ or tissue. *Ex vivo* methods can include, for example, the steps of harvesting cells from a subject, culturing the cells, transducing them with an expression vector, and maintaining the cells under conditions suitable for expression of the encoded FKBP11 peptide. These methods are known in the art of molecular biology. The transduction step can be accomplished by any standard means used for *ex vivo* gene therapy, including, for example, calcium phosphate, lipofection, electroporation, viral infection, and biolistic gene transfer.

Alternatively, liposomes or micro- and nanoparticles and polycations such as asialoglycoprotein/polylysine can be used. Cells that have been successfully transduced can be selected, for example, for expression of the coding sequence or of a drug resistance gene. The cells then can be lethally irradiated (if desired) and injected or implanted into the subject.

*In vivo* nucleic acid therapy can be accomplished by direct transfer of a functionally active DNA into mammalian somatic tissue or organ *in vivo.* Nucleic acids may also be administered *in vivo* by viral means. Nucleic acid molecules encoding an FKBP11 peptide may be packaged into retrovirus vectors using packaging cell lines that produce replication-defective retroviruses, as is well-known in the art. Other virus vectors may also be used, including recombinant adenoviruses and vaccinia virus, which can be rendered non-replicating. In addition to naked DNA or RNA, or viral vectors, engineered bacteria may be used as vectors.

The FKBP11 peptide may be administered alone, or in combination with other bioactive agents. Suitable bioactive agents include diabetes medications, which include insulin and insulin analogs, sulfonylureas, meglitinides, biguanides, thiazolidinediones, alpha-glucosidase inhibitors, or DPP-4 inhibitors. Sulfonylureas stimulate the beta cells of the pancreas to release more insulin. Chlorpropamide (Diabinese) is the only first-generation sulfonylurea still in use today. The second generation sulfonylureas are used in smaller doses than the first-generation drugs. There are three second-generation drugs: glipizide (Glucotrol and Glucotrol XL), glyburide (Micronase, Glynase, and Diabeta), and glimepiride (Amaryl). Meglitinides are drugs that also stimulate the beta cells to release insulin. Repaglinide (Prandin) and nateglinide (Starlix) are meglitinides. Metformin (Glucophage) is a biguanide. Biguanides lower blood glucose levels primarily by decreasing the amount of glucose produced by the liver. Rosiglitazone (Avandia) and pioglitazone (ACTOS) are in a group of drugs called thiazolidinediones. These drugs help insulin work better in the muscle and fat and also reduce glucose production in the liver. DPP-4 inhibitors help improve A1C without causing hypoglycemia. They work by preventing the breakdown of a naturally occurring compound in the body, GLP-1. GLP-1 reduces blood glucose levels in the body, but is broken down very quickly so it does not work well when injected as a drug itself. By interfering in the process that breaks down GLP-1, DPP-4 inhibitors allow it to remain active in the body longer, lowering blood glucose levels only when they are elevated. Sitagliptin (JANUVIA) and saxagliptin (ONGLYZA) are the two DPP-4 inhibitors currently on the market.

### Screening Assays

In general, candidate agents can be identified from large libraries of natural products or synthetic (or semi-synthetic) extracts or chemical libraries according to methods known in the art. Those skilled in the field of drug discovery and development will understand that the precise source of test extracts or compounds is not critical to the screening procedure(s).

Virtually any number of chemical extracts or compounds can be screened using the exemplary methods described herein. Examples of such extracts or compounds include, but are not limited to, plant-based, fungal-based, prokaryotic-based, or animal-based extracts, fermentation broths, and synthetic compounds, as well as modification of existing compounds. Numerous methods are also available for generating random or directed synthesis (e.g., semi-synthesis or total synthesis) of any number of chemical compounds, including, but not limited to, saccharide-based, lipid-based, peptide-based, polypeptide-based and nucleic acid-based compounds. Synthetic compound libraries and libraries of natural compounds in the form of bacterial, fungal, plant, and animal extracts are commercially available from a number of sources. In addition, natural and synthetically libraries can be produced, if desired, according to routine methods, e.g., by standard extraction and fractionation methods. Furthermore, if desired, any library or compound is readily modified using standard chemical, physical, or biochemical methods.

When a crude extract is found to have a desired activity, further fractionation of the positive lead extract may be necessary to isolate chemical constituents responsible for the observed effect. The goal of the extraction, fractionation, and purification process is the careful characterization and identification of a chemical entity within the crude extract having the desired activity. Assays can be used to purify the active component and to test derivatives thereof. Methods of fractionation and purification of such heterogenous extracts are known in the art. If desired, compounds shown to be useful agents for treatment are chemically modified according to methods known in the art. Compounds identified as being of therapeutic value may be subsequently analyzed using appropriate *in vitro* or animal models, for example, animal models of type 1 and/or type 2 diabetes.

Candidate agents encompass numerous chemical classes, but are most often organic molecules, e.g., small organic compounds having a molecular weight of more than 100 and less than about 2,500 daltons. Candidate agents contain functional groups necessary for structural interaction with proteins, particularly hydrogen bonding, and typically include at least an amine, carbonyl, hydroxyl or carboxyl group, for example, at least two of the functional chemical groups. The candidate agents often contain cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof.

For example, a microarray analysis on livers of mice that overexpress FKBP11 can be performed, providing information on FKBP11-mediated changes in gene expression in the liver. A similar methodology in an *in vitro* setting in which cells (preferably mammalian) are treated with candidate agents can be used to identify agents that induce similar gene expression patterns like FKBP11. Candidate agents that induce changes in gene expression that are similar to changes mediated by FKBP11, those candidates could be further tested for their potential effect on FKBP11 action/activity. In addition, potential candidates can be tested for their effect on glucose and insulin metabolism in *in vitro* or *in vivo* settings.

Cheminformatics and in-silico predictive models are used to increase the efficiency of the experimental approaches. Additional information such as compound - target interactions, target - mechanism of action/pathway relationships, and target -- disease associations can be mined from internal and publically available external databases. The combination of experimental and predicted compound-target pharmacological profiles can be used to prioritize compounds for additional screening and to provide evidence for proposed mechanisms of action. In addition, these profiles can be used to retrieve similar compounds for additional testing.

Chemogenomics library represents an additional opportunity to identify a biological target. To address the limitation of a suitable screening collection for use in phenotypic assays, the Chemogenomics screening collection was constructed in 2011. Chemogenomics sets consist of ∼5,000 compounds covering > 1,000 targets. Compounds screening set is created based on single targets or clustered biology space. These compound sets (10-20 compounds) provide an additional set of tools to confirm the biology space identified by their Chemogenomics screening hits.

Chemicals identified in addition to those already known to target the pathway should lead to additional compounds related in the targets or activity of the known compounds and these can be identified by the informatics tools. A significant portion of the screen will be a pathway enriched screen. Screening with compounds of known biological mechanism-of-action reduces transition time from the primary stage to a more focused screen based on improved selectivity and chemical properties.

Two strategies can be employed for compound selection. The first strategy is based on the identification of alternative targets from the bioinformatics screening to be performed. Compounds can be selected based on their selectivity profile, as well as chemical properties. The second strategy will select compounds following screening of compounds from focused chemical libraries, such as the chemogenomics set. This provides a library of up to 5000 compounds that covers ∼1000 biological targets for a full phenotypic screen. In combination with the bioinformatics results, appropriate compounds will be used for screening in the mouse model.

The use of chemoinformatics and *in silico* models can be employed to examine data from various studies. All of the compound efficacy data from screenings is mapped to targets and those targets used for a pathway-enrichment analysis. Component genes from pathways containing a significantly enriched number of screening hits can then be used to query the drug library. Compounds that target genes from the expanded pathways will then be selected for follow-up analysis in the animal models. The combination of experimental and predicted compound-target pharmacological profiles can be used to prioritize compounds for additional screening and to provide evidence for proposed mechanisms of action. In addition, these profiles can be used to retrieve similar compounds for additional testing.

The host cells described therein can be employed in a screening assay, to identify agents which upregulate/inhibit FKBP 11 activity within the context of glucose metabolism.

The present invention will be further understood by reference to the following non-limiting examples.

### Examples

### Example 1. Expression levels of FKBP11 in obese (ob/ob) and lean mice

### Materials and Methods

Livers were obtained from 6hr fasted lean and ob/ob mice. Livers were rapidly snap-frozen in liquid nitrogen and stored at -80C until further processing. For protein isolation, small pieces of liver (∼100mg) were homogenized in tissue lysis buffer. FKBP11 protein expression in liver lysates was determined using western blot analysis. For RNA isolation, small pieces (∼50mg) of liver were homogenized in QIAzol reagent (Qiagen). RNA was isolated using chloroform extraction and subsequent isopropanol precipitation. cDNA was produced using iScript cDNA synthesis kit (Biorad). Gene expression was analyzed by QPCR using SYBR green reagent and iCycler instrument. Relative gene expression levels were determined using delta Ct method.

### Results

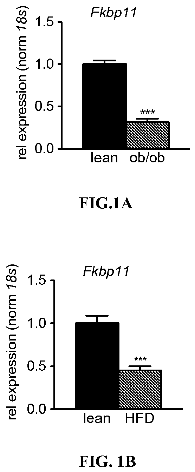

Figures 1A-1B show that hepatic gene expression levels of FKBP11 are reduced in obese and type 2 diabetic mice and in a high fat diet (HFD)-induced obese and insulin resistant mice when compared to lean mice. A similar pattern was seen with protein expression levels.

### Example 2. Effect of restored FKBP11 expression on glucose tolerance, hepatic gluconeogenic activity and insulin sensitivity

### Materials and Methods

Mice were intravenously injected with control (adLacZ) or FKBP11-containing adenovirus via the tail vein. Body weight, food intake and blood glucose levels were measured every other day. Five days after injection, mice were subjected to a glucose tolerance test (GTT). Mice were fasted overnight. In the morning, mice were intraperitoneally injected with a bolus of glucose and blood glucose concentrations were measured in time using a Contour glucose meter (Bayer). Seven days after adenovirus injection, mice were subjected to an insulin tolerance test (ITT). Mice were fasted for 6hrs and subsequently intraperitoneally injected with a bolus of insulin. Blood glucose concentrations were measured in time using a Contour glucose meter (Bayer). Mice were killed after a 6hrs fast on day nine after adenovirus injection.

### Results

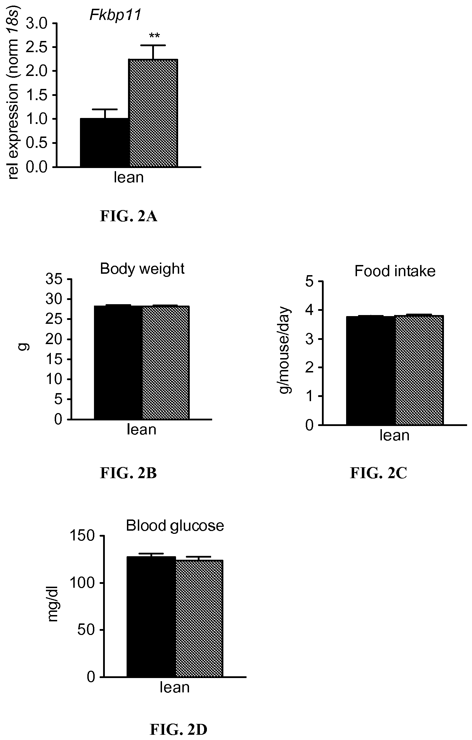

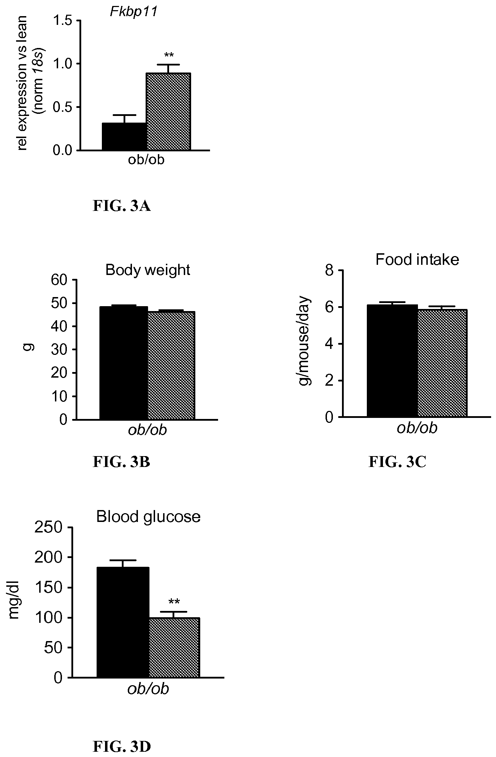

Overexpression of FKBP11 in livers of lean mice does not affect body weight (Fig 2B), food intake (Fig. 2C) and blood glucose levels (Fig. 2D). Similarly, overexpression of FKBP11 in livers of ob/ob mice does not affect body weight (Fig. 3C) or food intake (Fig. 3D), but it significantly lowers blood glucose levels (Fig. 3E) on ob/ob mice. By contrast, overexpression of FKBP 11 in livers of lean mice does not affect body weight (Fig 2B), food intake (Fig. 2C) and blood glucose levels (Fig. 2C).

### Example 3. The Effect of FKBP11 overexpression in an in vivo model of Type 1 diabetes

### Materials and Methods

Type I diabetes was induced by injecting C57B6/J mice with streptozotocin (STZ). Diabetes, as determined by glucose levels >500mg/dl develops within 4days. Mice that did not meet these criteria were not included in the study. Type 1 diabetic mice were intravenously injected with control (adLacZ) or FKBP11-containing adenovirus via the tail vein. Body weight, food intake and blood glucose levels were measured every other day.

### Results

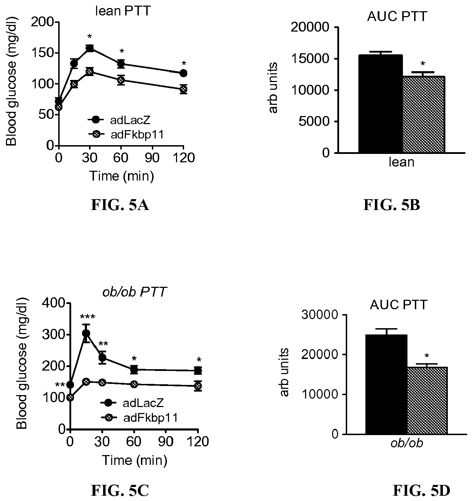

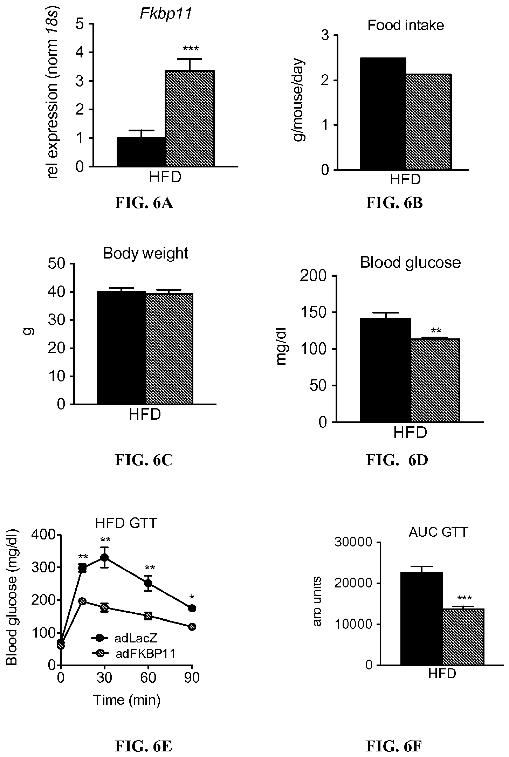

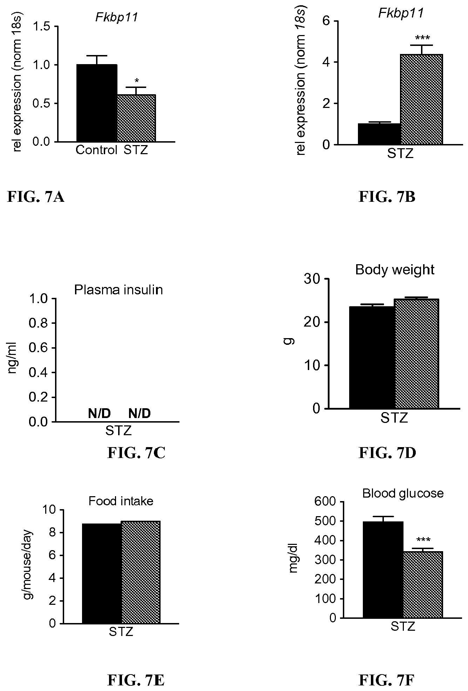

FKBP11 overexpression in HDF-fed and STZ-induced type 1 diabetic mice does not affect body weight (Fig. 6C and 7F) or food intake (Fig. 6B and 7E), but it significantly lowers blood glucose levels (Fig. 6E and 7F). Glucose tolerance (as assessed by glucose tolerance test GTT), hepatic gluconeogenic activity (as assessed by pyruvate tolerance test, PTT) and insulin sensitivity (as assessed by insulin tolerance, ITT), in ob/ob mice overexpressing FKBP11 are dramatically improved compared to mice that expressed a control virus (Figs. 4D- 4F and 5C-5D). FKBP11 overexpression in HDF-fed mice significantly improves glucose tolerance as assessed by GTT (Figs. 6E and F). By contrast, FKBP11 overexpression does not improve glucose tolerance or insulin sensitivity in lean mice (Fig. 4A- 4C) but it improves hepatic gluconeogenic activity (Fig. 5A and 5B).

FKBP11 overexpression does not affect insulin levels (Fig. 7C), body weight (Fig. 7D) or food intake (Fig. 7E) in streptozotocin (STZ)-induced type 1 diabetic mice.

### Example 4. Secretion of FKBP11 in lean and Obese mice/ MICE Overexpressing FKBP11

### Materials and Methods

Lean and ob/ob mice were killed by cardiac puncture under isoflurane anesthesia after a 6hr fast. Mice overexpressing LacZ or FKBP11 (adenovirus-mediated overexpression, intravenously injected via the tail vein) were killed by cardiac puncture under isoflurane anesthesia after a 6hr fast on day 4 after adenovirus injection. Blood was collected in heparin-coated tubes and centrifuged at 4degrees to obtain the plasma. Plasma was cleaned from albumin/IgG and loaded onto SDS gels. FKBP11 was visualized using western blot analysis. An ELISA was developed. Plates were coated with an FKBP11 antibody (raised in goat) and subsequently incubated with media from cells that overexpressed FKBP11. Bound FKBP11 was detected using a second FKBP11 antibody (raised in rabbit) and visualized using HRP labeled antibody and Turbo TMB ELISA reagent.

### Results

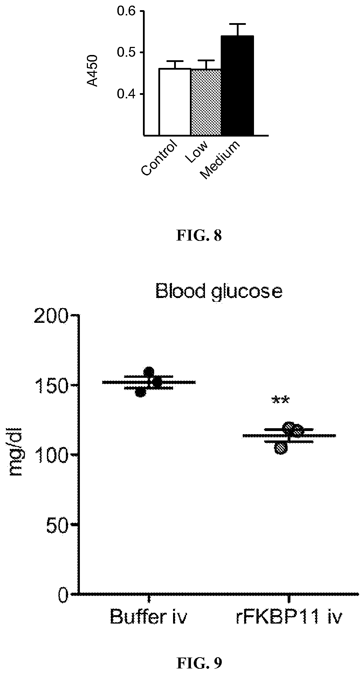

Mice that overexpress FKBP11 in the liver have higher plasma levels of FKBP 11 in as seen in studies using two different cohorts of mice) (data not shown). The presence of FKBP11 in cell culture medium was detected following FLAG tagged-FKBPl 1 expression and western blot analysis for FLAG in the cell culture media (data not shown). FKBP11 ELISA read outs (A450nm) from cell culture media of HEK cells overexpressing FKBP11 are presented in Fig. 8.

### Example 5. A single, intravenous injection of recombinant full-length FKBP11 reduces fasting blood glucose

### Materials and Methods

To induce obesity, wt mice (C57BL/6J) were fed a high-fat diet (45 kcal% fat) for six months. After establishment of obesity, mice were intravenously injected with 10 mg/kg recombinant FKBP11 (rFKBP11) or corresponding solvent via the tail vein. After an overnight (10PM-9AM) fast, blood glucose levels were measured using a Contour glucose meter (Bayer)

The results (Fig. 9) show that a single, intravenous injection of recombinant full-length FKBP11 reduces fasting blood glucose in obese and diabetic mice. This is further evidence of the potential importance of circulating FKBP11 in regulation of glucose metabolism.

### Discussion

Sulten, et al. Mamm. Genome, 17(4):322-331 (2006), reviewed the expression profile of FKBP19, and concluded that it suggests a unique role for FKBP19 in protein secretion. Other studies have identified FKBP11 as a potential marker for diagnosis of diabetes. For example, EP 1840573 lists FKBP11 as an example of a marker which could be used to diagnose a disease or a predisposition to a disease having a preinflammatory phase, for example, diabetes, before any clinical symptom of the disease is apparent. U.S. Patent Nos. 7,951,776 and 7,951,382, identify biological markers associated with the risk of developing diabetes, as well as methods of using such biological markers in diagnosis and prognosis of diabetes. FKBP11 is among the five hundred and forty eight (548) of the markers thus identified. Lu, et al., Mol. Cell. Prot., 7(8):1434-1450 (2008) describe a study associating 159 proteins (including FKBP11), with islet dysfunction. Lu, et al., disclose that FKBP11 and FKBP2, among many other proteins, are highly upregulated in the islets from a mouse model of insulin resistance.

By contrast, the studies described in this application show a direct link between low levels of secreted FKBP11 and glucose metabolism. The Examples show that FKBP11 is a crucial player in maintenance of glucose homeostasis in obese and type 2 diabetic mice as well as in a mouse model of type 1 diabetes. Hepatic expression levels of FKBP11 are reduced in obese and type 2 diabetic mice and in a high fat diet (HFD)-induced obese and insulin resistant mice when compared to lean mice (Fig. 1A-1B).

An adenoviral-mediated approach to restore FKBP11 expression in obese mice dramatically reduced both fasted blood glucose levels in obese mice.

Overexpression of FKBP11 in livers of ob/ob mice does not affect body weight (Fig. 3C) or food intake (Fig. 3D), but it significantly lowers blood glucose levels (Fig. 3E) on ob/ob mice. The same results were obtained with HDF-fed and STZ-induced type 1 diabetic mice that overexpress FKBP11. FKBP11 overexpression in HDF-fed and STZ-induced type 1 diabetic mice does not affect body weight (Fig. 6C and 7F) or food intake (Fig. 6B and 7E), but it significantly lowers blood glucose levels (Fig. 6D and 7F). By contrast, overexpression of FKBP11 in livers of lean mice does not affect body weight (Fig 2C), food intake (Fig. 2D) and blood glucose levels (Fig. 2E).

In addition, glucose tolerance (as assessed by glucose tolerance test GTT), hepatic gluconeogenic activity and insulin sensitivity in ob/ob mice overexpressing FKBP11 are dramatically improved compared to mice that expressed a control virus (Figs. 4D - 4F and 5C-5D). FKBP11 overexpression in HDF-fed mice significantly improves glucose tolerance as assessed by GTT (Figs. 6F and F). By contrast, FKBP11 overexpression does not improve glucose tolerance or insulin sensitivity in lean mice (Fig. 4A-4C) but it improves hepatic gluconeogenic activity (Fig. 5A and 5B).

FKBP11 overexpression does not affect insulin levels (Fig. 7E), body weight (Fig. 7D) or food intake (Fig. 7E) in streptozotocin (STZ)-induced type 1 diabetic mice.

Expression of FK-BP11 at high levels is not required for the effects described here. Rather, restoring FKBP11 expression levels to levels observed in lean healthy controls is sufficient to recover glucose tolerance and insulin sensitivity in obese mice.

These results confirm the biological significance of FKBP11 in regulation of glucose homeostasis, and provide important therapeutic potential for treatment of hyperglycemia in both type 1 and type 2 diabetes.

FKBP11 is predicted to reside in the ER membrane as a type 1 transmembrane protein. In addition to its broad tissue expression pattern, FKBP11 has detected in the circulation of mice. Significant levels of FKBP11 were detected in the circulation of mice. Further, FKBP11overexpressed in livers of lean mice was subsequently detected at significantly increased levels in the plasma of these mice (data not shown). FKBP11 is potentially cleaved in a yet unknown manner followed by secretion into the circulation where it might function as a hormone. Corresponding with the observation that obese mice have reduced hepatic FKBP11, plasma levels of FKBP11 also appear to be reduced in these mice. While not been bound by theory, secreted FKBP11 may be functioning as a hormone, regulating glucose metabolism; this provides numerous potential possibilities for the development of therapeutic interventions for the treatment of both type 2 and type 1 diabetes.

Accordingly, the present application also provides aspects according to the following numbered paragraphs:
1. A composition for increasing the levels of an FKBP11 peptide in a subject comprising an FKBP11 peptide or a nucleic acid encoding an FKBP11 peptide, wherein the FKBP11 peptide is selected from the group consisting of an FKBP11 polypeptide, a variant or a fragment thereof or a fusion protein containing an FKBP11 polypeptide, a variant or a fragment thereof.
2. The composition of paragraph 1, in a form selected from the group consisting of dry powders, tablets, wafers, films, lozenges, and capsules.
3. The composition of paragraph 1, in a form suitable for parenteral administration.
4. The composition of paragraph 1 wherein the FKBP11 polypeptide comprises SEQ ID NO: 1.
5. The composition of paragraph 1 comprising a nucleic acid encoding an FKBP11 polypeptide, a variant or a fragment thereof in a vector.
6. The composition of paragraph 5, wherein the vector is selected from the group consisting of bacteriophage, baculoviruses, tobacco mosaic virus, herpes viruses, cytomegalo virus, retroviruses, vaccinia viruses, adenoviruses, and adeno-associated viruses.
7. The composition of paragraph 1, wherein the FKBP11 peptide reduces blood glucose levels in the subject.
8. A method of increasing the levels of FKBP11 peptide in a subject comprising administering a composition comprising an FKBP11 peptide, or a nucleic acid encoding an peptide, wherein the FKBP11 peptide is selected from the group consisting of an FKBP11 polypeptide, a variant or a fragment thereof or a fusion protein containing an FKBP11 polypeptide, a variant or a fragment thereof.
9. The method of paragraph 8, wherein the wherein the subject is selected from a group consisting of a subject with type I diabetes, a subject with type II diabetes, an obese subject, a gestational diabetic, and a subject exhibiting insulin tolerance
10. The method of paragraph 8, wherein the FKBP11 peptide reduces blood glucose levels in the subject.
11. The method of paragraph 8, wherein the composition is in a form selected from the group consisting of dry powders, tablets, wafers, films, lozenges, and capsules.
12. The method of paragraph 8, wherein the composition is administered parenterally.
13. The method of paragraph 8, further comprising transforming one or more cells *ex vivo* to express the nucleic acid encoding an FKBP11 peptide and transferring the transformed cells into the subject.
14. The method of paragraph 13, wherein the cells are selected from the group consisting of pancreatic cells, islet cells and pancreatic precursor cells.
15. A kit comprising a first storage container and a second storage container, wherein the first storage contain comprises an FKBP11 peptide, or a nucleic acid encoding an FKBP11 peptide, and wherein the second storage container comprises an excipient, wherein the FKBP11 peptide is selected from the group consisting of an FKBP11 polypeptide, a variant or a fragment thereof or a fusion protein containing an FKBP11 polypeptide, a variant or a fragment thereof.
16. The kit of paragraph 15 wherein the first container is a cap and the second container is a vial the cap is secured to, and wherein the two containers are separated by a barrier.

## Claims

1. A composition for increasing the levels of an FKBP11 peptide in a subject comprising an FKBP11 peptide or a nucleic acid encoding an FKBP11 peptide, wherein the FKBP11 peptide is selected from the group consisting of an FKBP11 polypeptide, a variant or a fragment thereof or a fusion protein containing an FKBP11 polypeptide, a variant or a fragment thereof.

2. The composition of claim 1, in a form selected from the group consisting of dry powders, tablets, wafers, films, lozenges, and capsules.

3. The composition of claim 1, in a form suitable for parenteral administration.

4. The composition of claim 1 wherein the FKBP11 polypeptide comprises SEQ ID NO: 1.

5. The composition of claim 1 comprising a nucleic acid encoding an FKBP11 polypeptide, a variant or a fragment thereof in a vector.

6. The composition of claim 5, wherein the vector is selected from the group consisting of bacteriophage, baculoviruses, tobacco mosaic virus, herpes viruses, cytomegalo virus, retroviruses, vaccinia viruses, adenoviruses, and adeno-associated viruses.

7. The composition of claim 1, wherein the FKBP11 peptide reduces blood glucose levels in the subject.

8. A composition for use in a method of increasing the levels of FKBP11 peptide in a subject, wherein the composition comprises an FKBP11 peptide, or a nucleic acid encoding a peptide, wherein the FKBP11 peptide is selected from the group consisting of an FKBP11 polypeptide, a variant or a fragment thereof or a fusion protein containing an FKBP11 polypeptide, a variant or a fragment thereof.

9. The composition for use according to claim 8, wherein the wherein the subject is selected from a group consisting of a subject with type I diabetes, a subject with type II diabetes, an obese subject, a gestational diabetic, and a subject exhibiting insulin tolerance

10. The composition for use according to claim 8, wherein the FKBP11 peptide reduces blood glucose levels in the subject.

11. The composition for use according to claim 8, wherein the composition:
(a) is in a form selected from the group consisting of dry powders, tablets, wafers, films, lozenges, and capsules; or
(b) is administered parenterally.

12. The composition for use in a method according to claim 8, wherein the method comprises transforming one or more cells *ex vivo* to express the nucleic acid encoding an FKBP11 peptide and transferring the transformed cells into the subject.

13. The composition for use according to claim 12, wherein the cells are selected from the group consisting of pancreatic cells, islet cells and pancreatic precursor cells.

14. A kit comprising a first storage container and a second storage container, wherein the first storage contain comprises an FKBP11 peptide, or a nucleic acid encoding an FKBP11 peptide, and wherein the second storage container comprises an excipient, wherein the FKBP11 peptide is selected from the group consisting of an FKBP11 polypeptide, a variant or a fragment thereof or a fusion protein containing an FKBP11 polypeptide, a variant or a fragment thereof.

15. The kit of claim 14 wherein the first container is a cap and the second container is a vial the cap is secured to, and wherein the two containers are separated by a barrier.
